(19) Europäisches Patentamt
European Patent Office
Office européen des brevets

(11) EP 2 010 005 B1

(12) EUROPEAN PATENT SPECIFICATION

(45) Date of publication and mention of the grant of the patent:
**11.11.2009 Bulletin 2009/46**

(21) Application number: **06725210.6**

(22) Date of filing: **21.03.2006**

(51) Int Cl.:
*A23K 1/16* (2006.01) *A23K 1/18* (2006.01)
*A61K 31/197* (2006.01) *A61P 43/00* (2006.01)

(86) International application number:
**PCT/EP2006/060926**

(87) International publication number:
**WO 2007/107184 (27.09.2007 Gazette 2007/39)**

(54) **TREATMENT OF POULTRY FOR REDUCING THE FEED CONVERSION RATE OR FOR REDUCING THE INCIDENCE OF ASCITES**

BEHANDLUNG VON GEFLÜGEL ZWECKS REDUZIERUNG DER FUTTERUMWANDLUNGSRATE BZW. REDUKTION DES AUFTRETENS VON ASCITES

TRAITEMENT DE VOLAILLE POUR RÉDUIRE LE TAUX DE CONVERSION D'ALIMENTS OU POUR RÉDUIRE L'INCIDENCE DE L'ASCITE

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**07.01.2009 Bulletin 2009/02**

(73) Proprietor: **Taminco N.V.**
**9000 Gent (BE)**

(72) Inventors:
- **JANSSENS, Geert**
  **B-9270 Laarne (BE)**
- **KALMAR, Isabelle**
  **B-9000 Gent (BE)**
- **ROOSE, Peter**
  **B-9831 Sint-Martens-Latem (BE)**
- **SEGERS, Steven**
  **B-9550 Herzele (BE)**
- **VANNESTE, Piet**
  **B-9400 Denderwindeke (BE)**

(74) Representative: **Van Reet, Joseph et al**
**Gevers & Vander Haeghen,**
**Holidaystraat 5**
**1831 Diegem (BE)**

(56) References cited:
**WO-A-99/04784** **WO-A-03/011047**
**GB-A- 999 394** **US-A- 5 895 788**
**US-A1- 2002 182 276** **US-A1- 2004 156 882**

- **BALOG JANICE M: "Ascites syndrome (pulmonary hypertension syndrome) in broiler chickens: Are we seeing the light at the end of the tunnel?" AVIAN AND POULTRY BIOLOGY REVIEWS, vol. 14, no. 3, 2003, pages 99-126, XP009076214 ISSN: 1470-2061**
- **VILLAR-PATINO GONZALO ET AL: "Effects of dietary supplementation with vitamin C or vitamin E on cardiac lipid peroxidation and growth performance in broilers at risk of developing ascites syndrome" AMERICAN JOURNAL OF VETERINARY RESEARCH, vol. 63, no. 5, May 2002 (2002-05), pages 673-676, XP009076247 ISSN: 0002-9645**
- **GENG AILIAN ET AL: "Effects of dietary L-carnitine and coenzyme Q10 supplementation on performance and ascites mortality of broilers." ARCHIVES OF ANIMAL NUTRITION. DEC 2004, vol. 58, no. 6, December 2004 (2004-12), pages 473-482, XP009076246 ISSN: 1745-039X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 010 005 B1

## Description

The present invention relates to a method for the non-therapeutic treatment of poultry for the purpose of reducing the conversion rate of the feed used to raise the poultry by means of a particular agent, to the therapeutic and second medical use of that agent to reduce the incidence of ascites, and to a feed for poultry containing an amount of that agent.

In the broiler industry for raising poultry, in particular chickens, and especially broilers, improvements and developments have been made essentially in the breeding technique for phyletic lines of broilers and in the rearing technique for increasing the growth rate of broilers. Much emphasis is put on the growth rate and the feed conversion rate in the method of rearing broilers. A high-calorie feed enables to achieve a lower feed conversion rate, i.e. a lower amount of feed is required to produce a certain amount of poultry, but has brought about some problems. The high growth rate of the poultry during the rearing period can for example no longer be followed by sufficient body metabolic functions such as the cardiac functions, etc., and the imbalance between these two has increased the death rate, thereby lowering the raising rate and the productivity to cause great economic damage to the broiler industry.

One essential cause of death of broilers from the imbalance between the growth rate and the development of pulmo-cardiac functions is the syndrome called "ascites". This syndrome is considered as one of the most important causes of death for broilers. On a world-wide basis the occurrence level of ascites in commercial meat chickens is estimated at 4.7% (Maxwell H.M., Robertson G.W. British Poultry Science 39,203-215 (1998)). The primary occurrence of this disease is a hypoxemic condition resulting from several factors. Hereby the hematocrite concentration increases leading to an increased blood viscosity which gives on its turn rise to a pulmonary hypertension and possibly right heart side failure. An immediate consequence of this condition is that the venal pressure increases, leading to liquid migration out of the blood vessel in the abdominal cavities. Broilers growing up in conditions stimulating high growth rates have a natural sensitivity for hypoxemia and ascites due to a combination of high oxygen demand (needed for the high growth) and the relatively underdeveloped cardio-respiratory systems of these animals. Other factors contributing to this condition of primary hypoxemia are an insufficient ventilation system, low temperature of the environment, breeding on high altitude and an energy rich feed type (Herenda D.C., Franco D.A. Iowa State University Press, Iowa, p. 4-9 (1996)).

Another problem is that nowadays feed compositions for poultry are more and more supplemented with fats from vegetal sources to reduce the cost of the feed without compromising the total energy value of the feed for the animals. As a direct effect of this increased level of vegetal fat in the feed the oxidative stress for the animals increases and leads to higher mortality level.

In practice, it is of high economical importance to be able to decrease the feed conversion rate, i.e. the amount of feed required for 1 kg of body weight gain, without having to use a (more expensive) feed having a higher energy value. In this respect Fekete (Fekete S., Hegedüs M., Sós E. Magyar Állatorvosok Lapja 34(5), 311-314 (1978)) has done tests to verify whether it might be possible to reduce the feed conversion rate not by increasing the metabolizable energy value of the feed but by supplementing a poultry diet with pangamic acid (vitamin $B_{15}$), more particularly in an amount of 1000 mg per kg of feed. The results obtained by Fekete indicate however that the feed conversion rate was not affected by the pangamic acid and was for both the pangamic acid group and the control group equal to about 2.5.

As described above, a further important problem when raising broilers at high growth rates is the quite high incidence of ascites and the associated mortality. In the examples described in EP-B-0 981 967 the growth rate of the broilers was higher than 50 g/day. The high mortality which was due to ascites could be reduced considerably by adding coenzyme Q to the high-calorie pelletized feed. Coenzyme Q is however a complex molecule which is quite expensive to produce.

An object of the present invention is therefore to provide another agent which enables to reduce the incidence of ascites in poultry, in particular in poultry raised at a high growth rate.

A further object of the present invention is to provide a new treatment of poultry which enables to reduce the conversion rate of the feed used to raise the poultry, i.e. which enables to reduce the amount of feed required to produce a certain amount of poultry meat.

In a first aspect, the present invention concerns a method for the non-therapeutic treatment of poultry for the purpose of reducing the conversion rate of the feed used to raise the poultry, which treatment comprises orally administering a glycine compound to the poultry, which glycine compound corresponds to the following formula I or to a salt thereof:

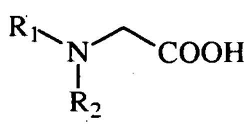

I

wherein $R_1$ and $R_2$ are independently an alkyl, an alkenyl or a hydroxyalkyl radical containing 1 to 18, preferably 1 to 6

carbon atoms or
wherein $R_1$ and $R_2$ form jointly together with the N atom a heterocyclic 5- or 6-membered ring.

In a second aspect, the present invention concerns a method for reducing the incidence of ascites in poultry by administering the same glycine compound thereto and the use of this compound for the manufacture of a medicament for reducing the incidence of ascites in poultry.

In a third aspect, the present invention concerns a feed for poultry which comprises at least 0.001 % by weight of said glycine compound.

In a preferred embodiment, the glycine compound is N,N-dimethylglycine (DMG), N,N-diethylglycine, N,N-diethanolglycine, N,N-dipropylglycine, N,N-diisopropylglycine, or a mixture and/or a salt thereof, the glycine compound being preferably DMG or a salt thereof.

N,N-Dimethylglycine (DMG) was first detected in 1938 as part of the active molecule pangamic acid (6-O-(dimethylaminoacetyl)-D-gluconic acid, PA) (Krebs E.T., Beord N.H., Malin R. Int. Red. Med. 164, 18 (1954)). The original report by Krebs declares that PA is always found together with the known B-vitamins, this fact together with the assigned biological functions were taken as reasons to account PA as vitamin $B_{15}$, although no disease state can as yet be attributed exclusively to a deficiency of the substance. Russian researchers reported that calcium pangamate could have a positive effect on the performance of athletes and on the cardiovascular and liver function. Further Russian studies showed that the pangamate formula has some influence on the restore of the immune system of guinea pigs and rats which were subjected to high intensity X-rays (Nizametidinova,G. Reports of the Kazan Veterinary Institute 112,100-104(1972)).

US-A-3 907 869 defines the component calcium pangamate as the ester of dimethylglycine and calcium gluconate and describes as advantages of PA the capability of enhancing oxidative metabolism is cells and tissues and eliminating the phenomena of hypoxia, as well as promoting lipid metabolism and acting as detoxicant.

As described in the article of Roger V. Kendall and John W. Lawson, "Recent Findings on N, N-Dimethylglycine (DMG): A new Nutrient for the New Millenium" (2000), calcium pangamate would not be stable under normal digestive processes and would rapidly hydrolyze to DMG when given after oral administration. When describing the known effects of DMG their article, these authors thus considered DMG as the active component of calcium pangamate.

In accordance with the present invention it has now been found that by orally administering DMG instead of pangamic acid to poultry, the feed conversion rate could be reduced (even after having eliminated any therapeutic effect of DMG) notwithstanding the fact that such a reduction of the feed conversion rate was not obtained by Fekete when administering pangamic acid to the poultry. This may possibly be explained by the fact that in the digestive system of poultry pangamic acid might not hydrolyze, or not sufficiently, to DMG. According to the present invention it has further been found that orally administering DMG to poultry considerably reduces the incidence of ascites and the associated mortality, in particular when the birds are under a higher metabolic stress. Although in the experiment of Fekete also a number of birds have died (were also disregarded when determining the feed conversion rate), he has not reported any effect of pangamic acid on the mortality. Based on the experiment of Fekete, the advantageous effects of the present invention on the feed conversion rate and on the incidence of ascites are thus quite surprising.

As mentioned hereabove, the present invention is concerned with a method for the non-therapeutic treatment of poultry for the purpose of reducing the conversion rate of the feed used to raise the poultry by orally administering a particular agent to the poultry, to a method or a second medical use of that agent for reducing the incidence of ascites in poultry and to a feed for poultry comprising that agent.

The invention is applicable to any type of commercial poultry operation but is primarily concerned with commercial chicken (broiler) and turkey growing operations. In a commercial chicken and turkey growing operation the flock is typically under substantial stress. As is well known, normal industry growing conditions include substantial density in the enclosure, for example, a density on the order of about 0.05 $m^2$ per chicken or turkey. Further, the ventilation in such commercial growing operations is often not a precisely controlled operation and the determination of appropriate ventilation including both heating and cooling is a very subjective operation. Further, the life span for a broiler ranges from about 40-60 days and the life span for a turkey ranges from 12-24 weeks so that the whole operation from birth to market in conditions under which growth is achieved is very stressful to the flock. Moreover, to aggravate the problem, growers will typically push the limits of recommended industry conditions which simply increases the stress on the flock.

Due to these growing conditions and the high growth rate, the occurrence level of ascites and the associated mortalily is already quite high in practice and limits the development of new feeds or production methods which causes even more metabolic or oxidative stress. A higher oxidative stress is for example obtained when the feed compositions contain more unsaturated fatty acids, for example more than 3 % by weight or more than 4 or even 5 % by weight of the feed, whilst a higher metabolic stress is obtained when the birds are made to take up more calories to increase the growth rate. These fatty acids are either free fatty acids or fatty acids bound for example in di- or triglycerides.

According to the present invention, it has been found that administration of appropriate and effective amounts of a glycine compound corresponding to formula (I) or to a salt thereof, for example a sodium or calcium salt, and especially DMG or a salt thereof, to poultry being grown under commercial growing operations enables to reduce the

incidence of ascites and the associated mortality rates in the flock. Further, it has been found that administration of such glycine compound or a salt thereof enables to reduce the feed conversion rate (in the healthy birds, i.e. without taking the advantageous effect on the mortality into account) and thus enables to use the feed more efficiently. Finally, the oxidative stress related to the presence of unsaturated fatty acids in the blood stream can be reduced.

The glycine compound administered to the poultry is preferably N,N-dimethylglycine (DMG), N,N-diethylglycine, N,N-diethanolglycine, N,N-dipropylglycine, N,N-diisopropylglycine, or a salt of these compounds, for example a sodium, potassium or calcium salt. The glycine compound may also comprise mixtures of these compounds and/or of the salts thereof. The most preferred glycine compound is DMG or a salt thereof.

When the glycine compound is water-soluble, such as DMG, it can be dosed in the drinking water of the poultry. Most preferably, the glycine compound is however administered via the feed of the poultry.

The basal diet to which the glycine compound is added can be any typical poultry diet meeting the nutritive needs of the broiler type bird, including starter, grower and finishing rations. A conventional diet includes selections among various protein, carbohydrate, vitamin and mineral sources and will generally contain about 12-25 % by weight crude protein, 0.5-10 % by weight crude fat and 2-12 % by weight crude fiber. The feed preferably has a crude protein content of at least 18.5 % by weight, a crude fat content of at least 4 % by weight, a starch content of at least 30 % by weight, and/or a crude fibre content of less than 5 % by weight.

The primary component is generally grain and processed grain by-products which supply carbohydrates and some protein. Protein meals from soybeans, alfalfa, corn gluten, cottonseed, sunflowers and other plants are often used to supply additional protein to the diet, as are animal by-products. Poultry feed compositions are generally supplemented with various vitamins and minerals (usually in the form of a premix to which the glycine compound can also be added), and molasses and animal fats are added to improve palatability and to increase or balance energy levels. They have generally a moisture content of less than 15 % by weight and preferably of less than 14 % by weight. General reference is made to National Research Council, Nutrient Requirements of Poultry. Nutrient Requirements of Domestic Animals. National Academy of Science, Washington, D.C. (1994), for a discussion of poultry nutrient requirements and typical poultry rations for various species and life phases of poultry, said reference being incorporated.herein.

The feed according to the invention has preferably a metabolizable energy value of at least 11.5 MJ/kg, and more preferably.of at least 12.0 MJ/kg, the energy value of the feed being preferably smaller than 14 MJ/kg, and more preferably smaller than 13.5 MJ/kg. By means of such a high-calorie feed, high growth rates can be achieved.

The metabolizable energy as referred to herein is obtained by subtracting the amount of energy as discharged in faeces and urine (waste calorie) from the amount of total energy of the intake feed (total calorie), and ordinary calorimetry may be applied to each feed composition to actually obtain the metabolizable energy of the composition. In general, the metabolizable energy value of feed for chickens is obtained on the basis of known tables of calorie components for it, and such known tables may be employed herein to obtain the metabolizable energy in question. The metabolizable energy value can more particularly be calculated by means of the following formula:

$$AME\ (MJ/kg) = 15.5CP + 34.3EE + 16.7ST + 13.0Su$$

Wherein: CP = crude protein
EE = ether extract (= crude fat)
St = starch
Su = sugars.

(see Larbier M, Declerq B, (1992) Nutrition et Alimentation des Volailles. INRA, Paris).

The standard (AOAC International) analysis methods are:

- dry matter: drying
- crude protein: Kjeldahl method
- crude ash: incineration
- crude fat: ether extraction (Sohxlet method)
- crude fibre: Fibertec analysis
- starch and sugars: Luff-Schoorl analysis (polarimetry).
  (see Official Methods of Analysis of AOAC International, 16 th ed. The Association of Official Analytical Chemists, Arlington, VA).

In a preferred embodiment of the invention, the preferred additive range of the glycine compound in the finished feed is about 0.001 to 0.5 % by weight, preferably about 0.005 to 0.1 % by weight. There is no evidence that use of the

higher amounts would cause any toxicity problems in treated poultry; however, the considerations of cost may become significant.

In the method according to the invention, the glycine compound is preferably administrated to the poultry for 7 days or longer, preferably for 14 days or longer, while they are of 10 to 35-days age. The poultry is preferably selected and raised in such a manner that over the period during which the glycine compound is administered the feed conversion rate is smaller than 2.50, preferably smaller than 2.45 and more preferably smaller than 2.40 kg feed/kg body weight gain and/or that during this period the growth rate of the poultry is higher than 50 g/day, and preferably higher than 60 g/day. The glycine compound it thus preferably administered to birds which are under metabolic stress so that the effects of the glycine compound administration are more pronounced.

Experimental results

The setup of the experiments described below is to assess the influence of DMG on:

- Performance in model species for fowl: mortality, feed rate conversion (FRC)
- Incidence of ascites: PCV, gross necropsy lesions
- Plasma metabolites: triglycerides (TG), non-esterified fatty acid (NEFA)

Methodology and materials

To examine all the effects associated with the supplementation of dimethylglycine in fowl feed, a test was setup with broilers as model species. In this slaughter test with 64 broiler hens, 14-day old chickens were bred for 26 days, i.e. till the age of 40 days. At the beginning of the test the broilers were ad random divided in 16 groups of 4 specimens. The birds were fed with one type of feed from the first day on till the end of the test period. The type of feed was attributed on a random basis to the different housings. The animals were setup with a colored ring around their leg for identification purposes within each housing. The control feed was based on a commercial broiler crumble enriched with 5% corn oil to increase the oxidative stress in the feed. Further 1% celite was mixed into the feed as external marker to allow the determination of the apparent nutrient digestibility. The feed had the following composition: 87.65% dry matter, 6.13% ash, 18.05% crude protein, 9.13% crude fat, 4.32% crude fibre and 50.01% other carbohydrates (including 89% starch and 2% sugar). It has a (calculated) metabolizable energy value of about 13.4 MJ/kg.

In the second type of feed 167 mg of DMG was added per kilogram to the same composition of feed as described for the control feed. Over the entire test period, the broilers consumed on average about 25 mg DMG per bird per day. The housing of the broilers consisted out of circular housings with an open roof and bottom and with a total surface of $0.72m^2$. The material of the fencing was a flexible wire netting with a height of 1 m and mazes of 2x2 cm and thickness of 2 mm. The bottom was strewed with a thick layer of turf with on top a layer of wood-shavings. Feed and water were ad libitum available at all times. The average environmental temperature was maintained at 15°C to increase the occurrence of ascites (Shlosberg A., Zadikov I., Bendheim U., Handji V., Berman E. Avian Pathology, 21,369-382 (1992)). A conventional light scheme of 23 hours light :1 hour dark was applied

Each bird was blood sampled and weighted 3 times, namely at days 1, 15 and 26. The hematocrite concentration was immediately determined by means of an ultracentrifugation on a sub sample of each blood sample. Next, the blood samples were subjected to a centrifugation and the blood plasma was kept at -20°C. On this plasma the concentrations of the plasma metabolites: triglycerines and free fatty acid (NEFA: non-esterified fatty acids) were determined.

The daily growth during the two periods (between the first and fifteenth day and between the sixteenth and twentysixth day) was calculated for each surviving animal. The feed uptake for each housing was also measured during both periods. The commercial feed conversion rate was calculated for each of the housings for both periods (FCRI and FCR II) and for the total growth period. This commercial feed conversion rate was calculated by dividing the total feed consumption by the weight gain of the birds which stayed alive during the entire experiment, including also the feed consumption of the birds that died. To determine only the non-therapeutic effect of DMG on the feed conversion rate, the actual feed conversion rate of the living birds was also calculated more particularly on the basis of weight gain and the feed consumed by these living birds (based on the weight gain of the birds that died, the amount of feed consumed by these birds was detracted from the total feed consumption).

At the start of the second period a sample of 100 g of manure was collected. The coefficients for the apparent metabolizability of macronutrients and the apparent nitrogen retention was calculated based on the external marker method with the acid insoluble (celite) as external marker

$$\textit{Apparent metabolizability} = 1 - ( NF / NV \times IV / IF )$$

*wherein : NF : percentage of examined nutrient in the faeces sample*
*NV : percentage of examined nutrient in the feed sample*
*IV : percentage of the indicator in the feed sample*
*IF : percentage of the indicator in the faeces sample*

To determine the above parameters both the faeces and the feed samples were analysed on the dry matter, crude ash, crude protein, crude fat and crude fibre (AOAC, 1980). The carbohydrates were calculated from the difference between the dry matter content and the rest of the macronutrients as determined by means of the Weende analysis. The content of insoluble ashes (celite) was determined according to the procedure of Atkinson et al. (1984).

Finally there was also an autopsy on all chickens. First, the carcass weight was determined by dissection of the head, the paws, evisceration and the deduction of the average feather weight. Next, the weight of the breast, buttock and thigh muscle were determined. Also the the heart, liver and abdominal fat were measured. Macroscopic lesions related to ascites were visually reviewed and described as indicated by Scheele et al. (2003) (Scheele, C.W., Van Der Klis, J.D., Kwakernaak,C., Buys, N., Decuypere, E., British Poultry Science, 44(3), 484-489 (2003)), these were: accumulation of liquid in the abdomen, a hydropericard and right heart dilatation. The latter was quantified by the ascites heart index (AHI) being the ratio of the the dry weight of the right ventricle to the dry weight of both ventricles after freeze drying.

Results

Ascites was the major cause of death during the test period of 26 days. The mortality and occurrence of ascites were substantially lower in the DMG supplemented group (Table 1 and 2). Next to a massive accumulation of fluid in the abdomen, all these animals showed a distinct right heart ventricle dilatation. At the beginning of the second phase of the experiment the chickens fed with the DMG supplementation showed a higher apparent metabolizability of the dry matter. From the calculated metabolic coefficients for the dry matter and the proteins it can be seen that the ability to metabolise both dry matter and proteins seems to be improved in the DMG supplemented group (Table 3). From the second period on, the DMG supplemented group also showed a significantly improved feed conversion and also an increased growth rate. The total growth rate was however substantially not affected so that the improved feed conversion has to be explained by a lower feed consumption.

*Table 1: Influence of oral DMG supplementation on the production performance indicators of broilers.*

| | DMG | Control |
|---|---|---|
| Mortality (%) | 6.25 | 9.38 |
| Ascites (%) | 6.25 | 15.63 |
| Apparent dry matter metabolizability | 65.05 | 59.59 |
| Growth rate (g/d) | | |
| Phase I (days 1-15) | 70 | 73 |
| Phase II (days 15-26) | 87 | 80 |
| Total (days 1-26) | 77 | 76 |
| Commercial feed conversion | | |
| FCR I (days 1-15) | 2.23 | 2.23 |
| FCR II (days 15-26) | 2.17 | 2.33 |
| FCR total (days 1-26) | 2.19 | 2.26 |
| Actual feed conversion | | |
| FCR I (days 1-15) | 2.07 | 2.05 |
| FCR II (days 15-26) | 2.13 | 2.33 |
| FCR total (days 1-26) | 2.09 | 2.16 |

*Table 2: Ascites heart index for in autopsy examined hearts of the birds*

| | DMG | Control | Influence DMG (%) |
|---|---|---|---|
| AHI, all animals | 0.242 | 0.294 | -17.5 |

(continued)

| | DMG | Control | Influence DMG (%) |
|---|---|---|---|
| AHI, surviving animals | 0.241 | 0.299 | -19.2 |

*Table 3: Metabolic coefficients for both dry matter and proteins*

| | DMG | Control |
|---|---|---|
| Metabolic ability dry matter (%) | 91.5 | 89.4 |
| Metabolic ability protein (%) | 73 | 69.3 |

*Table 4: Influence of oral DMG supplementation on the blood parameters of broilers.*

| | DMG | Control |
|---|---|---|
| Hematocrite value (vol%) | | |
| beginning | 28.3 | 27.2 |
| after 15 days | 31.6 | 30.6 |
| after 25 days | 31.3 | 30.3 |
| Triglycerine value (mg/dl) | | |
| beginning | 73 | 90 |
| after 15 days | 101 | 95 |
| after 25 days | 51 | 38 |
| NEFA value (mg/dl) | | |
| beginning | 0.29 | 0.25 |
| after 15 days | 0.49 | 0.48 |
| after 25 days | 0.60 | 1.49 |

In the above experiment, the actual occurrence of the broiler ascites syndrome of 4.7% (Maxwell H.M., Robertson G.W. British Poultry Science 39,203-215 (1998)) was significantly increased by the setup of the test. The inventors could clearly show that there was a significant difference in the occurrence of ascites in respectively the DMG supplemented and control group of animals. Hence, it could be concluded that DMG has a protective effect on the pathogenesis of ascites. This effect was also confirmed in the increased hematocrite values for the DMG supplied group of animals. The direct proof of the positive effect of DMG on the ascites condition was found during the autopsy tests on the hart of the chickens. For all animals the AHI (Ascites Heart Index)) was calculated as the ration of the dry weight of the right heart ventricle to the dry weight of both heart ventricles. This parameter gives a clear indication on the occurrence of right heart hypertrophy (symptom of ascites syndrome). Significantly higher AHI values were found for the group of chickens who weren't supplemented with DMG.

Not only was the ascites syndrome positively influenced by DMG, it was further found that the DMG supplementation led to a reduced feed conversion rate, especially during the second growing period. The commercial feed conversion rate, which included the therapeutic effect of DMG on ascites and which is very important from a practical point of view was clearly improved. However, also the actual feed conversion rate, calculated only on the basis of the weight gain and of the feed consumed by the birds which stayed alive, was clearly improved. (The same actual feed conversion rate can also be calculated on the basis of total weight gain of all animals, including the birds that died during the trial). This proves that DMG has also a non-therapeutic effect on the feed conversion rate. This non-therapeutic effect was moreover coupled to an increase of dry matter and protein metabolizability for the chickens fed with the DMG supplemented diet.

In the blood panel of the animals, substantial differences in the concentration of triglycerides and significant differences in the concentration of free fatty acids (NEFA) were detected. The decrease of free fatty acids, with the DMG supplemented group, can be explained by either an increased extraction of these products from the blood or by the fact that less NEFA were mobilized from the fat reserves of the animals. Since DMG also gives rise to an increase in apparent metabolizability of the feed, it is most likely that the reduced values for the NEFA were due to the fact that there were less NEFA mobilized from the fat reserves of the chickens. This conclusion can be further supported by the high levels

of triglycerides detected in the blood samples of the DMG supplemented group. This leads to the conclusion that DMG can be important as support for the energy metabolism and the reduction of metabolic stress.

In summary, it was found that the supplementation of 0.001-0.5 wt. % (based on the feed) of the glycine compound, more particularly of DMG or its salts, has a beneficial effect on a disease in fowl called ascites. The DMG supplementation leads to an increase of the hematocrite level so that the organism of the bird can deal more effectively with limited oxygen supply to the tissues. Moreover the DMG also plays a role in the reduction of the free fatty acid content in the blood stream which can lead due to the presence of unsaturated bonds in the molecular structure to oxidative stress leading to the death of the animal. This property is important in the modern feed formulation technology which uses more and more vegetal fats to supplement the feed. Further DMG has a distinct influence on both the commercial and the actual feed conversion, an important economic parameter in the cultivation of fowl in general. This feed conversion is directly linked to the effect that a DMG supplementation has on the apparent metabolizability of both dry matter and proteins. In general it was found that DMG has a positive influence on the occurrence of ascites in fowl and on the bird's energy metabolism.

## Claims

1. A method for the non-therapeutic treatment of poultry for the purpose of reducing the conversion rate of the feed used to raise the poultry, which treatment comprises orally administering at least one glycine compound to the poultry, which glycine compound corresponds to the following formula (I) or to a salt thereof:

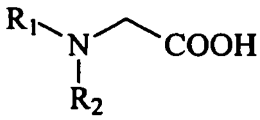

(I)

wherein $R_1$ and $R_2$ are independently an alkyl, an alkenyl or a hydroxyalkyl radical containing 1 to 18, preferably 1 to 6 carbon atoms or wherein $R_1$ and $R_2$ form jointly together with the N atom a heterocyclic 5- or 6-membered ring.

2. The method according to claim 1, wherein the glycine compound is selected from the group consisting of N,N-dimethylglycine (DMG), N,N-diethylglycine, N,N-diethanolglycine, N,N-dipropylglycine, N,N-diisopropylglycine, or mixtures or salts thereof, the glycine compound being preferably DMG or a salt thereof.

3. The method according to claim 1 or 2, wherein the glycine compound is administered via the drinking water of the poultry.

4. The method according to any one of the claims 1 to 3, wherein the glycine compound is administered via said feed.

5. The method according to any one of the claims 1 to 4, wherein the poultry comprises broiler chickens.

6. The method according to any one of the claims 1 to 5, wherein the glycine compound is administered during a period to poultry which is selected and raised in such a manner that over said period the actual feed conversion rate is smaller than 2.50, preferably smaller than 2.45 and more preferably smaller than 2.40 kg feed/kg body weight gain and/or in such a manner that over said period the growth rate of the poultry is higher than 50 g/day, and preferably higher than 60 g/day.

7. The method according to any one of the claims 1 to 6, wherein the glycine compound thereof is administered in an amount of between 0.001 and 0.5 % by weight of said feed, preferably in an amount of between 0.005 and 0.1 % by weight of said feed.

8. A feed for poultry comprising at least 0.001 % by weight, preferably at least 0.005 % by weight of a glycine compound, which glycine compound corresponds to the following formula (I) or to a salt thereof:

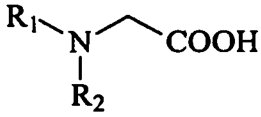

(I)

wherein $R_1$ and $R_2$ are independently an alkyl, an alkenyl or a hydroxyalkyl radical containing 1 to 18, preferably 1 to 6 carbon atoms or wherein $R_1$ and $R_2$ form jointly together with the N atom a heterocyclic 5- or 6-membered ring.

**9.** The feed according to claim 8, wherein the glycine compound is selected from the group consisting of N,N-dimethylglycine (DMG), N,N-diethylglycine, N,N-diethanolglycine, N,N-dipropylglycine, N,N-diisopropylglycine, or mixtures or salts thereof, the glycine compound being preferably DMG or a salt thereof.

**10.** The feed according to claim 8 or 9, which comprises said glycine compound in an amount of between 0.001 and 0.5 % by weight, preferably in an amount of between 0.005 and 0.1 % by weight.

**11.** The feed according to any one of the claims 8 to 10, which has a metabolizable energy value of at least 11.5 MJ/kg, and preferably of at least 12.0 MJ/kg, the energy value of the feed being preferably smaller than 14 MJ/kg, more preferably smaller than 13.5 MJ/kg.

**12.** The feed according to any one of the claims 8 to 11, which has a crude protein content of at least 18.5 % by weight, a crude fat content of at least 4 % by weight, a starch content of at least 30 % by weight, and/or a crude fibre content of less than 5 % by weight.

**13.** The feed according to any one of the claims 8 to 12, which has an unsaturated fatty acid content of at least 3 % by weight, preferably of at least 4 % by weight and more preferably of at least 5 % by weight.

**14.** The feed according to any one of the claims 8 to 13, which has a moisture content of less than 15 % by weight, preferably of less than 14 % by weight.

**15.** A glycine compound for use in a method for reducing the incidence of ascites in poultry, the glycine compound corresponding to the following formula (I) or to a salt thereof:

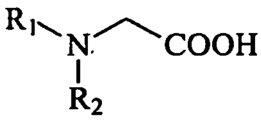

(I)

wherein $R_1$ and $R_2$ are independently an alkyl, an alkenyl or a hydroxyalkyl radical containing 1 to 18, preferably 1 to 6 carbon atoms or wherein $R_1$ and $R_2$ form jointly together with the N atom a heterocyclic 5- or 6-membered ring.

**16.** The glycine compound according to claim 15, wherein the glycine compound is selected from the group consisting of N,N-dimethylglycine (DMG), N,N-diethylglycine, N,N-diethanolglycine, N,N-dipropylglycine, N,N-diisopropylglycine, or mixtures or salts thereof, the glycine compound being preferably DMG or a salt thereof.

**17.** The glycine compound according to claim 15 or 16, wherein the glycine compound is added to poultry feed, the resultant feed comprising said glycine compound in an amount of between 0.001 and 0.5 % by weight, preferably in an amount of between 0.005 and 0.1 % by weight.

**18.** The glycine compound according to any one of the claims 15 to 17, wherein the glycine compound is administered during a period to poultry which is selected and raised in such a manner that over said period the actual feed conversion rate is smaller than 2.50, preferably smaller than 2.45 and more preferably smaller than 2.40 kg feed/kg body weight gain and/or in such a manner that over said period the growth rate of the poultry is higher than 50 g/day, and preferably higher than 60 g/day.

**Patentansprüche**

**1.** Ein Verfahren zur nicht therapeutischen Behandlung von Geflügel zwecks Reduzierung der Umwandlungsrate des zur Aufzucht des Geflügels verwendeten Futters, wobei die Behandlung die orale Verabreichung zumindest einer Glycinverbindung an das Geflügel umfasst, wobei die Glycinverbindung der folgenden Formel (I) oder einem Salz davon entspricht:

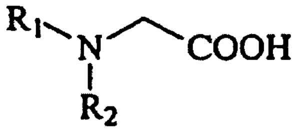

(I)

wobei $R_1$ und $R_2$ unabhängig ein Alkyl-, ein Alkenyl- oder ein Hydroxyalkylradikal mit 1 bis 18, vorzugsweise 1 bis 6 Kohlenstoffatomen sind oder wobei $R_1$ und $R_2$ zusammen mit dem N-Atom einen heterozyklischen 5- oder 6-gliedrigen Ring formen.

**2.** Das Verfahren nach Anspruch 1, wobei die Glycinverbindung aus der Gruppe bestehend aus N,N-Dimethylglycin (DMG), N,N-Diethylglycin, N,N-Diethanolglycin, N,N-Dipropylglycin, N,N-Diisopropylglycin, oder Mischungen oder Salzen davon ausgewählt wird, wobei die Glycinverbindung vorzugsweise DMG oder ein Salz davon ist.

**3.** Das Verfahren nach Anspruch 1 oder 2, wobei die Glycinverbindung über das Trinkwasser des Geflügels verabreicht wird.

**4.** Das Verfahren nach jedem der Ansprüche 1 bis 3, wobei die Glycinverbindung über das erwähnte Futter verabreicht wird.

**5.** Das Verfahren nach jedem der Ansprüche 1 bis 4, wobei das Geflügel Masthühner umfasst.

**6.** Das Verfahren nach jedem der Ansprüche 1 bis 5, wobei die Glycinverbindung eine Zeit lang Geflügel verabreicht wird, das so ausgewählt und aufgezogen wird, dass die tatsächliche Futterumwandlungsrate während des erwähnten Zeitraums geringer als 2,50, vorzugsweise geringer als 2,45 und noch besser geringer als 2,40 kg Futter/kg Körpergewichtszunahme ist und/oder so, dass die Wachstumsrate des Geflügels während der erwähnten Zeit höher als 50 g/Tag, und vorzugsweise höher als 60 g/Tag ist.

**7.** Das Verfahren nach jedem der Ansprüche 1 bis 6, wobei die Glycinverbindung daraus in einer Menge von zwischen 0,001 und 0,5 Gew.% des erwähnten Futters, vorzugsweise in einer Menge von zwischen 0,005 und 0,1 Gew.% des erwähnten Futters verabreicht wird.

**8.** Ein Futter für Geflügel, welches zumindest 0,001 Gew.%, vorzugsweise zumindest 0,005 Gew.% einer Glycinverbindung enthält, wobei die Glycinverbindung der folgenden Formel (I) oder einem Salz davon entspricht:

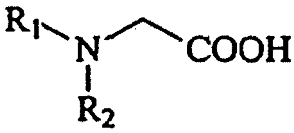

(I)

wobei $R_1$ und $R_2$ unabhängig ein Alkyl-, ein Alkenyl- oder ein Hydroxyalkylradikal mit 1 bis 18, vorzugsweise 1 bis 6 Kohlenstoffatomen sind oder wobei $R_1$ und $R_2$ zusammen mit dem N-Atom einen heterozyklischen 5- oder 6-gliedrigen Ring formen.

**9.** Das Futter nach Anspruch 8, wobei die Glycinverbindung aus der Gruppe bestehend aus N,N-Dimethylglycin (DMG), N,N-Diethylglycin, N,N-Diethanolglycin, N,N-Dipropylglycin, N,N-Diisopropylglycin, oder Mischungen oder Salzen davon ausgewählt wird, wobei die Glycinverbindung vorzugsweise DMG oder ein Salz davon ist.

**10.** Das Futter nach Anspruch 8 oder 9, welches die erwähnte Glycinverbindung in einer Menge von zwischen 0,001 und 0,5 Gew.%, vorzugsweise in einer Menge von zwischen 0,005 und 0,1 Gew.% enthält.

**11.** Das Futter nach jedem der Ansprüche 8 bis 10, das einen metabolisierbaren Energiewert von mindestens 11,5 MJ/kg, und vorzugsweise von mindestens 12,0 MJ/kg hat, wobei der Energiewert des Futters vorzugsweise geringer als 14 MJ/kg, noch besser geringer als 13,5 MJ/kg ist.

**12.** Das Futter nach jedem der Ansprüche 8 bis 11, das einen Rohproteingehalt von mindestens 18,5 Gew.%, einen Rohfettgehalt von mindestens 4 Gew.%, einen Stärkegehalt von mindestens 30 Gew.%, und/oder einen Rohfasergehalt von weniger als 5 Gew.% hat.

**13.** Das Futter nach jedem der Ansprüche 8 bis 12, das einen Gehalt an ungesättigten Fettsäuren von mindestens 3 Gew.%, vorzugsweise mindestens 4 Gew.% und noch besser mindestens 5 Gew.% hat.

**14.** Das Futter nach jedem der Ansprüche 8 bis 13, das einen Feuchtigkeitsgehalt von weniger als 15 Gew.%, vorzugsweise weniger als 14 Gew.% hat.

**15.** Eine Glycinverbindung zur Anwendung in einem Verfahren zwecks Reduzierung des Auftretens von Aszites bei Geflügel, wobei die Glycinverbindung der folgenden Formel (I) oder einem Salz davon entspricht:

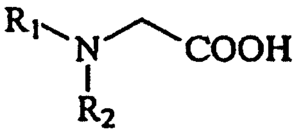

(I)

wobei $R_1$ und $R_2$ unabhängig ein Alkyl-, ein Alkenyl- oder ein Hydroxyalkylradikal mit 1 bis 18, vorzugsweise 1 bis 6 Kohlenstoffatomen sind oder wobei $R_1$ und $R_2$ zusammen mit dem N-Atom einen heterozyklischen 5- oder 6-gliedrigen Ring formen.

**16.** Die Glycinverbindung nach Anspruch 15, wobei die Glycinverbindung aus der Gruppe bestehend aus N,N-Dimethylglycin (DMG), N,N-Diethylglycin, N,N-Diethanolglycin, N,N-Dipropylglycin, N,N-Diisopropylglycin, oder Mischungen oder Salzen davon ausgewählt wird, wobei die Glycinverbindung vorzugsweise DMG oder ein Salz davon ist.

**17.** Die Glycinverbindung nach Anspruch 15 oder 16, wobei die Glycinverbindung dem Geflügelfutter zugesetzt ist, sodass das **dadurch** entstandene Futter die erwähnte Glycinverbindung in einer Menge von zwischen 0,001 und 0,5 Gew.%, vorzugsweise in einer Menge von zwischen 0,005 und 0,1 Gew.% enthält.

**18.** Die Glycinverbindung nach jedem der Ansprüche 15 bis 17, wobei die Glycinverbindung eine Zeit lang Geflügel verabreicht wird, das so ausgewählt und aufgezogen wird, dass die tatsächliche Futterumwandlungsrate während des erwähnten Zeitraums geringer als 2,50, vorzugsweise geringer als 2,45 und noch besser geringer als 2,40 kg Futter/kg Körpergewichtszunahme ist und/oder so, dass die Wachstumsrate des Geflügels während des erwähnten Zeitraums höher als 50 g/Tag, und vorzugsweise höher als 60 g/Tag ist.

## Revendications

**1.** Procédé pour le traitement non thérapeutique de volaille dans le but de réduire le taux de conversion des aliments pour élever la volaille, lequel traitement comprend l'administration orale d'au moins un composé de glycine à la volaille, lequel composé de glycine correspond à la formule (1) suivante ou à un sel de celui-ci :

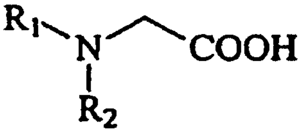

(I)

dans laquelle $R_1$ et $R_2$ sont indépendamment un radical alkyle, un radical alcényle ou un radical hydroalkyle contenant de 1 à 18, de préférence de 1 à 6 atomes de carbone ou dans laquelle $R_1$ et $R_2$ forment conjointement avec l'atome N un hétérocyclique à 5 ou 6 chaînons.

2. Procédé selon la revendication 1, dans lequel le composé de glycine est choisi dans le groupe consistant en N,N-diméthylglycine (DMG), N,N-diéthylglycine, N,N-diéthanolglycine, N,N-dipropylglycine, N,N-diisopropylglycine, ou des mélanges ou des sels de celles-ci, le composé de glycine étant de préférence de la DMG ou un sel de celle-ci.

3. Procédé selon la revendication 1 ou 2, dans lequel le composé de glycine est administré via l'eau potable de la volaille.

4. Procédé selon l'une quelconque des revendications 1 à 3, dans lequel le composé de glycine est administré via lesdits aliments.

5. Procédé selon l'une quelconque des revendications 1 à 4, dans lequel la volaille comprend des poulets à rôtir.

6. Procédé selon l'une quelconque des revendications 1 à 5, dans lequel le composé de glycine est administré pendant une période à la volaille qui est choisie et élevée de telle manière qu'au cours de ladite période, le taux effectif de conversion des aliments soit inférieur à 2,5, de préférence inférieure à 2,45 et mieux encore inférieur à 2,40 kg d'aliments / kg de gain de poids corporel et / ou de telle manière qu'au cours de ladite période, le taux de croissance de la volaille soit supérieur à 50 g / jour et de préférence supérieur à 60 g / jour.

7. Procédé selon l'une quelconque des revendications 1 à 6, dans lequel le composé de glycine est administré dans une quantité comprise entre 0,001 et 0,5 % en poids desdits aliments, de préférence dans une quantité comprise entre 0,005 et 0,1 % en poids desdits aliments.

8. Aliments pour volaille comprenant au moins 0,001 % en poids, de préférence au moins 0,005 % en poids d'un composé de glycine, lequel composé de glycine correspond à la formule (I) suivante ou à un sel de celui-ci :

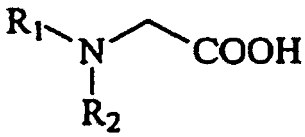

(I)

dans laquelle $R_1$ et $R_2$ sont indépendamment un radical alkyle, un radical alcényle ou un radical hydroalkyle contenant de 1 à 18, de préférence de 1 à 6 atomes de carbone ou dans laquelle $R_1$ et $R_2$ forment conjointement avec l'atome N un hétérocyclique à 5 ou 6 chaînons.

9. Aliments selon la revendication 8, dans lesquels le composé de glycine est choisi dans le groupe consistant en N, N-diméthylglycine (DMG), N,N-diéthylglycine, N,N-diéthanolglycine, N,N-dipropylglycine, N,N-diisopropylglycine, ou des mélanges ou des sels de celles-ci, le composé de glycine étant de préférence de la DMG ou un sel de celle-ci.

10. Aliments selon la revendication 9, qui comprennent ledit composé de glycine dans une quantité comprise entre 0,001 et 0,5 % en poids, de préférence dans une quantité comprise entre 0,005 et 0,1 % en poids.

11. Aliments selon l'une quelconque des revendications 8 à 10, qui ont une valeur énergétique métabolisable d'au moins 11,5 MJ / kg, et de préférence d'au moins 12,0 MJ / kg, la valeur énergétique des aliments étant de préférence inférieure à 14 MJ / kg, mieux encore inférieure à 13,5 MJ / kg.

12. Aliments selon l'une quelconque des revendications 8 à 11, qui ont une teneur en protéines brutes d'au moins 18,5 % en poids, une teneur en graisse brute d'au moins 4 % en poids, une teneur en amidon d'au moins 30 % en poids et / ou une teneur en fibres brutes de moins de 5 % en poids.

13. Aliments selon l'une quelconque des revendications 8 à 12, qui ont une teneur en acides gras insaturés d'au moins 3 % en poids, de préférence d'au moins 4 % en poids et mieux encore d'au moins 5 % en poids.

14. Aliments selon l'une quelconque des revendications 8 à 13, qui ont une teneur en humidité de moins de 15 % en

poids, de préférence de moins de 14 % en poids.

**15.** Composé de glycine à utiliser dans un procédé destiné à réduire l'incidence de l'ascite dans la volaille, le composé de glycine correspondant à la formule (I) suivante ou à un sel de celui-ci :

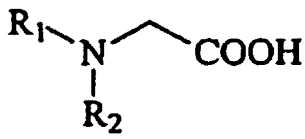

(I)

dans laquelle $R_1$ et $R_2$ sont indépendamment un radical alkyle, un radical alcényle ou un radical hydroalkyle contenant de 1 à 18, de préférence de 1 à 6 atomes de carbone ou dans laquelle $R_1$ et $R_2$ forment conjointement avec l'atome N un hétérocyclique à 5 ou 6 chaînons.

**16.** Composé de glycine selon la revendication 15, le composé de glycine étant choisi dans le groupe consistant en N, N-diméthylglycine (DMG), N,N-diéthylglycine, N,N-diéthanolglycine, N,N-dipropylglycine, N,N-diisopropylglycine, ou des mélanges ou des sels de celles-ci, le composé de glycine étant de préférence de la DMG ou un sel de celle-ci.

**17.** Composé de glycine selon la revendication 15 ou 16, le composé de glycine étant ajouté aux aliments de la volaille, les aliments résultants comprenant ledit composé de glycine dans une quantité comprise entre 0,001 et 0,5 % en poids, de préférence dans une quantité comprise entre 0,005 et 0,1 % en poids.

**18.** Composé de glycine selon l'une quelconque des revendications 15 à 17, le composé de glycine étant administré pendant une période à la volaille qui est choisie et élevée de telle manière qu'au cours de ladite période, le taux effectif de conversion des aliments soit inférieur à 2,5, de préférence inférieure à 2,45 et mieux encore inférieur à 2,40 kg d'aliments / kg de gain de poids corporel et / ou de telle manière qu'au cours de ladite période, le taux de croissance de la volaille soit supérieur à 50 g / jour et de préférence supérieur à 60 g / jour.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0981967 B
- US 3907869 A

### Non-patent literature cited in the description


- **Maxwell H.M. ; Robertson G.W.** *British Poultry Science,* 1998, vol. 39, 203-215
- **Fekete S. ; Hegedüs M. ; Sós E.** *Magyar Állatorvosok Lapja,* 1978, vol. 34 (5), 311-314
- **Krebs E.T. ; Beord N.H. ; Malin R.** *Int. Red. Med.,* 1954, vol. 164, 18
- **Nizametidinova,G.** *Reports of the Kazan Veterinary Institute,* 1972, vol. 112, 100-104
- **Roger V. Kendall ; John W. Lawson.** *Recent Findings on N, N-Dimethylglycine (DMG): A new Nutrient for the New Millenium,* 2000
- **Poultry.** Nutrient Requirements of Domestic Animals. National Academy of Science, 1994
- **Larbier M ; Declerq B.** *Nutrition et Alimentation des Volailles.,* 1992
- **Shlosberg A. ; Zadikov I. ; Bendheim U. ; Handji V. ; Berman E.** *Avian Pathology,* 1992, vol. 21, 369-382
- **Scheele, C.W. ; Van Der Klis, J.D. ; Kwakernaak,C. ; Buys, N. ; Decuypere, E.** *British Poultry Science,* 2003, vol. 44 (3), 484-489